# EUROPÄISCHE PATENTSCHRIFT

(11) **EP 1 970 001 B1**
(45) Veröffentlichungstag und Bekanntmachung des Hinweises auf die Patenterteilung: **23.07.2014**
(21) Anmeldenummer: 07005469.7
(22) Anmeldetag: 16.03.2007
(51) Int. Cl.: A61B 5/0215, A61B 5/03, A61M 5/172

(54) **Katheter mit Drucksensorik**
Catheter with pressure sensoring
Cathéter doté d'un capteur de pression

(43) Veröffentlichungstag der Anmeldung: 17.09.2008
(73) Patentinhaber: Brainlab AG, 85622 Feldkirchen (DE)
(72) Erfinder: Mittermeyer, Stephan, 84036 Landshut (DE); Hartlep, Andreas, 83629 Weyarn (DE)
(74) Vertreter: Schwabe - Sandmair - Marx

(56) Entgegenhaltungen:
- US-A- 5 427 114
- US-A- 5 701 905
- US-A- 5 704 352
- US-A- 6 019 728
- US-A1- 2004 060 362
- US-A1- 2005 215 945
- US-A1- 2006 287 569
- US-B1- 6 454 720

## Beschreibung

Die Erfindung betrifft einen Katheter zur Verabreichung einer Substanz in ein Körpergewebe, insbesondere in Gehirnstrukturen. Speziell beschäftigt sich die Erfindung mit Kathetern zur Platzierung in festem Gewebe für die konvektionsunterstützte Verabreichung von Substanzen. Bei der genannten konvektionsunterstützten Verabreichung von Substanzen wird eine Substanz, beispielsweise ein Medikament, mit Hilfe eines positiven Druckgradienten in ein Gewebe eingebracht.

Um den Katheter platzieren zu können, wird entweder ein Katheter aus einem relativ starren Material verwendet, oder ein Stilett aus einem starren Material (z.B. Edelstahl) wird in den Katheter für die Platzierung eingebracht. Ein positionsgenaues Setzen des Katheters ist äußerst wichtig für den Behandlungserfolg; deshalb sollte die vorbestimmte bzw. geplante Trajektorie eingehalten werden.

Es kommt jedoch immer wieder vor, dass Katheter platziert werden, die von der geplante Trajektorie abweichen, und zwar auf Grund der Festigkeitseigenschaften der vorliegenden anatomischen Strukturen, insbesondere aufgrund vorhandener Oberflächen (wie z.B. Sulci) oder wegen inhomogener Eigenschaften des Gehirngewebes (wie z.B. unterschiedlicher Elastizitäten). Damit eine zuverlässige und vorhersagbare Verteilung, insbesondere eine Verteilung, die vorab computerunterstützt simuliert wird, möglich wird, muss aber die geplante Trajektorie eingehalten werden.

Es ist grundsätzlich bekannt, mit Hilfe von Kathetern Drücke in Gewebeabschnitten zu messen, beispielsweise aus der US 5,987,995 und der US 6,120,457. Hier steht die Messung des Druckes, der an einem bestimmten Punkt im Gewebe oder im Körper vorliegt, im Vordergrund.

Aus der US 5,704,352 ist ein Katheter mit einem Drucksensor bekannt, wobei der Drucksensor voneinander beabstandete Sonden aufweist, mittels derer der Fluiddruck im Lumen des Katheters gemessen wird.

Es ist die Aufgabe der Erfindung, einen Katheter zur Verabreichung einer Substanz in ein Körpergewebe, insbesondere in Gehirnstrukturen, so auszugestalten, dass die Verteilung der durch den Katheter eingebrachten Substanz zuverlässig und vorhersagbar bleibt. Insbesondere sollen Formänderungen des Katheters beim Einbringen in das Gewebe detektierbar werden.

Diese Aufgabe wird durch einen Katheter gemäß dem Anspruch 1 gelöst. Die Unteransprüche definieren bevorzugte Ausführungsformen der Erfindung.

Bei einem erfindungsgemäßen Katheter mit einem länglichen Katheterkörper, der ein Lumen umgibt, ist im Katheterkörper eine von Drucksensor-Anordnung vorgesehen, mittels der eine Druckverteilung über einen Bereich des Katheters erfasst wird. Mit anderen Worten weist der Katheter an seinem in das Körpergewebe eindringenden Ende (distales Ende) eine Drucksensorik auf, die den Druckzustand des Katheters selbst erfasst, nämlich den Druck im Kathetermaterial. Es wird also der Katheterzustand erfasst, indem ein Druckzustand ermittelt wird.

In diesem Kontext erlaubt die vorliegende Erfindung ein Verfahren durchzuführen, um den Material-Druckzustand des Katheters mit Hilfe einer Drucksensorik zu ermitteln, oder eine Verwendung einer Drucksensorik zur Ermittlung des Druckzustandes in einem Katheter.

Wenn die Druckverteilung über einen Bereich des Katheters erfasst werden kann, wird es erfindungsgemäß möglich, auch die Verformung des Katheters zu bestimmen. Wenn ein Katheter zu einer Seite hin gebogen bzw. gestaucht wird, wird sich auf der Innenseite (auf der konkaven Seite) ein höherer Druck im Material ergeben, während das Material auf der Außenseite (konvexe Seite) einen niedrigeren Materialdruck aufweist. Durch die Drucksensor-Anordnung und die Auswertung der aus ihr erhaltenen Informationen lässt sich deshalb eine Richtungsinformation für die Biegung des Katheters ermitteln, und der Verwender erhält bei der Auswertung dieser Daten eine sofortige Rückmeldung darüber, dass der Katheter gerade die geplante Trajektorie verlässt. Es stehen dann mehrere Optionen zur Verfügung, nämlich einerseits ein neues Setzen des Katheters, andererseits eine Anpassung des Verabreichungsplanes an die reale Position, an welcher der Katheter die Substanz verabreicht. In diesem Zusammenhang kann die Verabreichung mit dem neuen Verabreichungspunkt oder -gebiet nochmals computerunterstützt simuliert werden, und zielführende Anpassungen sind möglich. Solche Anpassungen können einerseits Anpassungen der Substanz-Strömungsrate sein, andererseits kann der Katheter noch etwas weiter nach vorne oder etwas zurückgesetzt werden, um die Verteilung der Substanz in der gewünschten Weise zu garantieren.

Der Bereich des Katheters, über den die Druckverteilung erfasst wird, kann unterschiedlich beschaffen sein. Er kann einen Bereich um einen einzigen Drucksensor herum umfassen oder einen Bereich um mehrere Drucksensoren herum. Die Drucksensoren-Anordnung umfasst also mindestens einen, insbesondere aber mehrere Drucksensoren.

Gemäß einer Ausführungsvariante können Drucksensoren oder eine Teilgruppe von Drucksensoren im Wesentlichen entlang eines Längenabschnitts des Katheters angeordnet sein, insbesondere in vorbestimmten oder gleichmäßigen Abständen. Stattdessen oder zusätzlich besteht die Möglichkeit, Drucksensoren oder eine Teilgruppe von Drucksensoren im Wesentlichen in einer Querschnittsebene des Katheters anzuordnen, insbesondere umfänglich im Katheterkörper zu verteilen. Ferner können Drucksensoren oder eine Teilgruppe von Drucksensoren am distalen Ende oder über eine Länge am distalen Ende des Katheters angeordnet werden.

Die Drucksensoren können verschiedenartige, für den Einsatzfall geeignete Ausführungen haben, insbesondere piezoelektrische Elemente umfassen, Dehnmessstreifen-Elemente umfassen, oder - ganz allgemein -, auf Druck- oder Längenänderungen reagierende elektrische Widerstandselemente umfassen. Zur Weiterleitung der von den Drucksensoren ermittelten Daten werden erfindungsgemäß insbesondere Schnittstellen und/oder Signalabgriffe bzw. Signalweiterleitungseinrichtungen an den Drucksensoren angeordnet, beispielsweise gedruckte, aufgedampfte oder im Kathetermaterial eingelassene, dünne Leiterbahnen.

Wenn piezoelektrische Drucksensoren verwendet werden, die als integrierte Komponenten bereitgestellt werden, kann die Druckmessung auf eine Spannungsmessung zurückgeführt werden, weil die gemessene Spannung am piezoelektrischen Drucksensor direkt zu dem Grad der Biegung bzw. der Druckveränderung im Verhältnis steht. Hier lässt sich dann auch eine quantitative Aussage über die Intensität bzw. den Grad der Biegung treffen.

Die Erfindung wird im Weiteren anhand von Ausführungsformen und unter Bezugnahme auf die beiliegenden Zeichnungen näher erläutert. Sie kann alle hierin beschriebenen Merkmale einzeln sowie in jedweder sinnvollen Kombination umfassen und als Vorrichtung, als Verfahren oder als Verwendung definiert werden. In den beiliegenden Zeichnungen zeigen:
- Figur 1: eine schematische Darstellung eines erfindungsgemäßen Katheters mit Drucksensorik;
- Figur 2: einen Schnitt in der Ebene A-A in Figur 1;
- Figur 3: eine prinzipielle Darstellung der Druckerfassung;

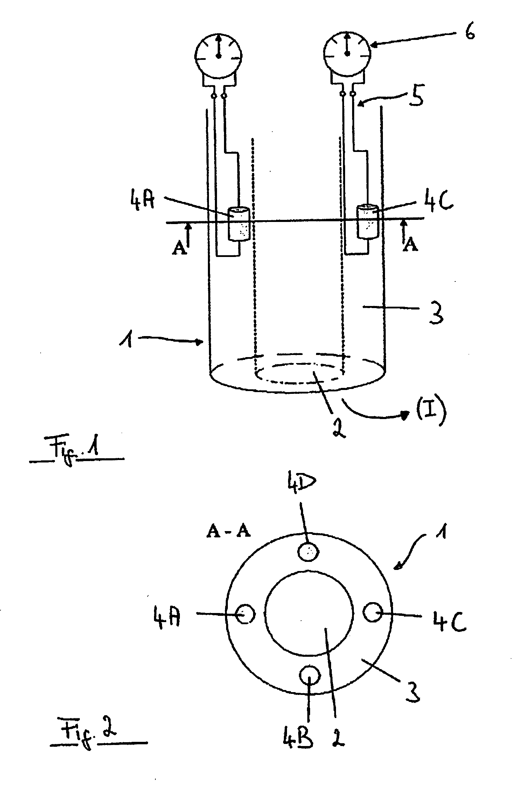

Eine schematische Darstellung einer Ausführungsform eines erfindungsgemäß ausgestalteten Katheters ist den Figuren 1 und 2 zu entnehmen, wobei die Figur 2 eine Schnittdarstellung an der Ebene A-A in Figur 1 ist. Der Katheter ist insgesamt mit dem Bezugszeichen 1 bezeichnet, und er weist einen Katheterkörper 3 sowie ein von dem Katheterkörper 3 umschlossenes Lumen 2 auf, durch welches eine Substanz, beispielsweise ein Medikament, in ein Körpergewebe infundiert wird.

In den Wandungen des Katheterkörpers sind bei dieser Ausführungsform Drucksensoren eingebracht, und in der Darstellung der Figur 1 sind die Drucksensoren 4A und 4C zu sehen. Die Drucksensoren 4A und 4C stehen beispielsweise schematisch für Piezokristall-Elemente, welche bei Verformungen (in der Längsachse) elektrische Spannungssignale abgeben. Diese Spannungssignale werden an den Drucksensoren abgegriffen und durch elektrische Leiterbahnen (z.B. gedruckte Leiterbahnen oder dünne Metallfasern) an Messeinrichtungen geleitet, von denen eine in Figur 1 das Bezugszeichen 6 trägt. Der Figur 2 ist noch zu entnehmen, dass bei der gezeigten Anordnung insgesamt vier Drucksensoren vorhanden sind, nämlich die Drucksensoren 4A, 4B, 4C und 4D, wobei die Sensoren regelmäßig umfänglich verteilt sind, also hier einen Abstand von 90° haben.

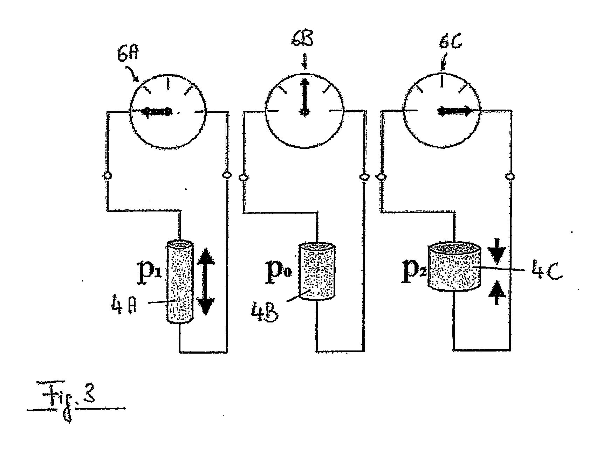

Wird nun, beispielsweise beim Setzen des Katheters in ein Gehirngewebe, der Katheter verbogen, kann dies beispielsweise in einer Richtung geschehen, die in Figur 1 mit dem gebogenen Pfeil (1) angezeigt ist. Wenn dies geschieht, wird sich der Druck im Kathetermaterial auf der Seite des Sensors 4C durch die Materialkomprimierung erhöhen; andererseits wird der Druck auf der Seite des Sensors 4A abfallen, weil das Material sich hier ausdehnt. Eine Situation für den Druck nach einer solchen Biegung (I) ist in Figur 3 dargestellt, und zwar für die Sensoren 4A, 4B und 4C.

Für den Drucksensor 4A wird ein Druck p₁ gemessen, der niedriger ist als der Druck p₀ an 4B, weil der Drucksensor 4B in der neutralen Zone liegt. Der Druck p₂ am Drucksensor 4C ist wiederum höher, weil das Kathetermaterial dort zusammengedrückt wird. Die Messeinrichtungen zeigen diese unterschiedlichen Drücke an, und dies ist schematisch mit 6A, 6B und 6C gekennzeichnet. Aus einem solchen Druckverlauf lässt sich dann qualitativ schließen, dass der Katheter in Richtung des Pfeiles (I) gebogen worden ist; und aus der jeweiligen abgegriffenen Größe (Spannungswert am Piezo-Drucksensor 4A, 4B, 4C, 4D) lässt sich sogar eine quantitative Aussage über die Biegung machen.

## Patentansprüche

1. Katheter (1) zur Verabreichung einer Substanz in ein Körpergewebe, insbesondere in Gehirnstrukturen, mit einem länglichen Katheterkörper (3), der ein Lumen (2) umgibt, wobei im Katheterkörper (3) eine Drucksensor-Anordnung (4A-4D) vorgesehen ist, mittels der eine Druckverteilung über einen Bereich des Katheters (1) erfasst wird, wobei die Drucksensor-Anordnung (4) im Katheterkörpermaterial eingebracht ist und den Druckverlauf im Katheterkörper misst.

2. Katheter nach Anspruch 1, **dadurch gekennzeichnet, dass** Drucksensoren oder eine Teilgruppe von Drucksensoren (4) im Wesentlichen in einer Querschnittsebene des Katheters (1) angeordnet sind, insbesondere umfänglich im Katheterkörper verteilt sind.

3. Katheter nach Anspruch 1 oder 2, **dadurch gekennzeichnet, dass** Drucksensoren oder eine Teilgruppe von Drucksensoren im Wesentlichen entlang eines Längenabschnitts des Katheters (1) angeordnet sind, insbesondere in vorbestimmten oder gleichmäßigen Abständen.

4. Katheter nach einem der Ansprüche 1 bis 3, **dadurch gekennzeichnet, dass** Drucksensoren (4A-4D) oder eine Teilgruppe von Drucksensoren am distalen Ende oder über eine Länge am distalen Ende des Katheters (1) angeordnet sind.

5. Katheter nach einem der Ansprüche 1 bis 4, **dadurch gekennzeichnet, dass** die Drucksensoren piezoelektrische Elemente umfassen.

6. Katheter nach einem der Ansprüche 1 bis 5, **dadurch gekennzeichnet, dass** die Drucksensoren Dehnmessstreifen-Elemente umfassen

7. Katheter nach einem der Ansprüche 1 bis 6, **dadurch gekennzeichnet, dass** die Drucksensoren auf Druck- oder Längenänderungen reagierende elektrische Widerstandselemente umfassen.

8. Katheter nach einem der Ansprüche 1 bis 7, **dadurch gekennzeichnet, dass** an den Drucksensoren Schnittstellen und/oder Signalabgriffe bzw. Signalweiterleitungseinrichtungen angeordnet sind.

## Claims

1. A catheter (1) for administering a substance into a body tissue, in particular into brain structures, comprising an elongated catheter body (3) which surrounds a lumen (2), wherein a pressure sensor array (4A-4D) is provided in the catheter body (3), by means of which a pressure distribution is detected over a range of the catheter (1), wherein the pressure sensor array (4) is introduced into the material of the catheter body and measures the pressure profile in the catheter body.

2. The catheter according to Claim 1, **characterised in that** pressure sensors or a partial group of pressure sensors (4) are arranged substantially in a cross-sectional plane of the catheter (1), in particular distributed circumferentially in the catheter body.

3. The catheter according to Claim 1 or 2, **characterised in that** pressure sensors or a partial group of pressure sensors are arranged substantially along a longitudinal portion of the catheter (1), in particular at predetermined or uniform distances.

4. The catheter according to any one of Claims 1 to 3, **characterised in that** pressure sensors (4A-4D) or a partial group of pressure sensors are arranged at the distal end or over a length at the distal end of the catheter (1).

5. The catheter according to any one of Claims 1 to 4, **characterised in that** the pressure sensors include piezoelectric elements.

6. The catheter according to any one of Claims 1 to 5, **characterised in that** the pressure sensors include resistive wire strain elements.

7. The catheter according to any one of Claims 1 to 6, **characterised in that** the pressure sensors include electrical resistance elements which respond to changes in pressure or length.

8. The catheter according to any one of Claims 1 to 7, **characterised in that** interfaces and/or signal taps or signal relaying means are arranged on the pressure sensors.

## Revendications

1. Cathéter (1) destiné à administrer une substance dans un tissu corporel, en particulier dans des structures cérébrales, comportant un corps de cathéter allongé (3) entourant une lumière (2), dans lequel est prévu un ensemble de capteurs de pression (4A à 4D) dans le corps de cathéter (3) au moyen duquel une répartition de pression est détectée sur une zone du cathéter (1), dans lequel l'ensemble de capteurs de pression (4) est positionné dans le matériau du corps de cathéter et mesure l'évolution de la pression dans le corps de cathéter.

2. Cathéter selon la revendication 1, **caractérisé en ce que** des capteurs de pression ou un sous-ensemble de capteurs de pression (4) sont agencés sensiblement dans un plan transversal du cathéter (1) et sont en particulier répartis dans le corps de cathéter de manière circonférentielle.

3. Cathéter selon la revendication 1 ou 2, **caractérisé en ce que** des capteurs de pression ou un sous-ensemble de capteurs de pression sont agencés sensiblement le long d'une partie longitudinale du cathéter (1), en particulier à des distances prédéterminées ou égales.

4. Cathéter selon l'une des revendications 1 à 3, **caractérisé en ce que** des capteurs de pression (4A à 4D) ou un sous-ensemble de capteurs de pression sont agencés à des extrémités distales ou sur une certaine longueur à l'extrémité distale du cathéter (1).

5. Cathéter selon l'une des revendications 1 à 4, **caractérisé en ce que** les capteurs de pression incluent des éléments piézoélectriques.

6. Cathéter selon l'une des revendications 1 à 5, **caractérisé en ce que** les capteurs de pression incluent des éléments extensométriques.

7. Cathéter selon l'une des revendications 1 à 6, **caractérisé en ce que** les capteurs de pression incluent des éléments de résistance électrique qui réagissent à des variations de pression ou de longueur.

8. Cathéter selon l'une des revendications 1 à 7, **caractérisé en ce que** des interfaces et/ou des prises de signaux ou des dispositifs de propagation de signaux sont agencés sur les capteurs de pression.
